# FASCICULE DE BREVET EUROPEEN

(11) **EP 4 055 074 B1**
(45) Date de publication et mention de la délivrance du brevet: **18.02.2026**
(21) Numéro de dépôt: 20816284.2
(22) Date de dépôt: 06.11.2020
(51) Int. Cl.: C08G 18/32, C08G 63/672

(54) **RÉSINE BIOSOURCÉE POUR UNE COMPOSITION COSMÉTIQUE ET SON PROCÉDÉ DE FABRICATION**
BIOBASIERTES HARZ FÜR EINE KOSMETISCHE ZUSAMMENSETZUNG UND VERFAHREN ZUR PRODUKTION DAVON
BIO-BASED RESIN FOR A COSMETIC COMPOSITION, AND PROCESS FOR THE PRODUCTION THEREOF

(30) Priorité: 08.11.2019 FR 1912564
(43) Date de publication de la demande: 14.09.2022
(73) Titulaire: ROQUETTE FRERES, 62136 Lestrem (FR); CENTRE NATIONAL DE LA RECHERCHE SCIENTIFIQUE (CNRS), 75016 Paris (FR); Université de Lille, 59000 Lille (FR); Centrale Lille Institut, 59650 Villeneuve-d'Ascq (FR); École Nationale Supérieure de Chimie, 59650 Villeneuve d'Ascq (FR); Université d'Artois, 62000 Arras (FR); Fiabila, 28130 Maintenon (FR); Mader Composites France, 84700 Sorgues (FR)
(72) Inventeur: SAINT-LOUP, René, 59160 LOMME (FR); RATAJ, Véronique, 59710 Pont-à-Marcq (FR); ZINCK, Philippe, 59170 Croix (FR); POUPON, Andy, 29520 Saint-Goazec (FR); NOUGUEREDE, Olivier, 28130 Maintenon (FR); ROUSSEL, Joël, 84700 Sorgues (FR); LAUBÉ, Florian, 31130 QUINT-FONSEGRIVES, (FR)
(74) Mandataire: Plasseraud IP
(86) Numéro de dépôt international: PCT/FR2020/052025
(87) Numéro de publication internationale: WO 2021/089963

(56) Documents cités:
- EP-A1- 2 923 735
- WO-A1-2015/165902
- US-B2- 10 457 771

## Description

### Domaine technique

La présente invention concerne une résine polyester biosourcée comprenant un mélange de diols et de polyacides, son procédé de préparation ainsi qu'une composition filmogène et un vernis à ongles comprenant ladite résine. La résine polyester peut notamment être utile en tant que liant dans un vernis à ongles.

### Technique antérieure

Aujourd'hui, les grands groupes de cosmétique et les consommateurs désirent des vernis à ongles totalement biosourcés. Toutefois, il existe actuellement très peu de résines biosourcées pouvant jouer le rôle de liant dans un vernis à ongles.

On connait, par exemple, un polyester copolymère de 1,2-propanediol, d'acide citrique, d'acide succinique et d'acide laurique commercialisé sous la référence Natipol^{®} 1303 par Mäder.

Toutefois, cette résine n'est pas aussi performante que les résines pétrosourcées, notamment en termes de brillance, d'adhésion, de stabilité, de dureté et de temps de séchage.

Il existe donc un besoin en une résine biosourcée comme substitut aux résines pétrosourcées pour obtenir un vernis à ongles ayant des performances adéquates et qui est en outre plus respectueux de l'environnement.

Le brevet US 10 457 771 B2 décrit une résine polyester destinée à être utilisée comme revêtement de feuilles de métal.

### Résumé

L'invention a ainsi pour objet une résine polyester obtenue par polymérisation :
- d'un mélange de diols comprenant l'isosorbide et un diol ramifié en C₃-C₆ ; et
- d'un mélange de polyacides comprenant l'acide succinique et l'acide sébacique.

Un autre objet de l'invention est un procédé de préparation d'une résine polyester comprenant les étapes suivantes :
i) réaction d'isosorbide et d'un polyacide choisi parmi l'acide succinique ou l'acide sébacique ;
ii) réaction du mélange obtenu à l'étape i) avec un diol ramifié en C₃-C₆ et un polyacide choisi parmi l'acide succinique ou l'acide sébacique,
dans lequel le polyacide utilisé dans l'étape i) est différent du polyacide utilisé dans l'étape ii).

Un autre objet de l'invention est une composition filmogène comprenant la résine polyester selon l'invention, un polymère filmogène cellulosique, et un solvant organique.

L'invention a également pour objet un vernis à ongles résine polyester selon l'invention, un polymère filmogène cellulosique, et un solvant organique, ainsi qu'un composé choisi parmi un plastifiant, une deuxième résine, un agent rhéologique, une matière colorante, un additif et les mélanges de ceux-ci.

Un autre objet de l'invention est l'utilisation de la résine polyester selon l'invention en tant que liant dans un vernis à ongles.

### Description des modes de réalisation

### Résine polyester

La présente invention concerne une résine polyester.

Par « résine polyester » on entend, au sens de la présente invention, un polymère comprenant des fonctions esters obtenu par polymérisation de diols et de polyacides.

Par « diol » on entend, au sens de la présente invention, un composé ayant deux fonctions hydroxyle.

Par « polyacide » on entend, au sens de la présente invention, un composé ayant au moins deux fonctions acide carboxylique.

Lorsque le terme « solvant organique » est utilisé au singulier dans la présente demande il désigne, sauf indication contraire, à la fois un seul solvant organique ainsi que des mélanges de solvants organiques.

La résine polyester de l'invention est obtenue par polymérisation d'un mélange d'au moins deux diols et d'un mélange d'au moins deux polyacides.

Le mélange de diols comprend l'isosorbide et un diol ramifié en C3-C6.

Selon un mode de réalisation particulier, le diol ramifié en C3-C6 est choisi parmi le 1,2-propanediol, le 1,3-butanediol, le 2,3-butanediol, le 1,2-pentanediol, et leurs mélanges. De préférence, le diol ramifié en C3-C6 est le 1,2-propanediol.

L'utilisation d'un diol ramifié en C3-C6 permet avantageusement d'obtenir une résine ayant une Tg supérieure à -30°C et une bonne solubilité dans les solvants utilisés pour les vernis à ongles.

Le mélange de diols peut en outre comprendre un diol additionnel, distinct de l'isosorbide et du diol ramifié en C3-C6. Selon un mode de réalisation particulier, le mélange de diols comprend en outre le 1,3-propanediol, le 1,4-butanediol, le 1,5-pentanediol, le 1,6-hexanediol et leurs mélanges.

Selon un mode de réalisation particulier, le mélange de diols consiste en l'isosorbide et un diol ramifié en C3-C6.

Selon un mode de réalisation particulier, la résine polyester ne contient pas de motifs obtenus par polymérisation d'un triol. Ainsi, les diols sont les seuls monomères hydroxylés utilisés dans la réaction de polymérisation avec les polyacides.

Le mélange de diols (isosorbide, diol ramifié en C3-C6 et éventuellement diol additionnel) peut notamment représenter de 30 à 65%, de préférence de 35 à 60%, plus préférentiellement de 40 à 55%, en moles des monomères incorporés dans la résine polyester.

L'isosorbide peut notamment représenter de 15 à 40%, de préférence de 20 à 35%, plus préférentiellement de 25 à 30%, en moles des monomères incorporés dans la résine polyester.

Le diol ramifié en C3-C6 peut notamment représenter de 10 à 35%, de préférence de 15 à 30%, plus préférentiellement de 20 à 25%, en moles des monomères incorporés dans la résine polyester.

Le ratio molaire entre l'isosorbide et le diol ramifié en C3-C6 incorporés dans la résine polyester peut notamment être supérieur ou égal à 1, en particulier de 1 à 2, plus particulièrement 1 à 1,5.

Le mélange de polyacides comprend l'acide succinique et l'acide sébacique.

Le mélange de polyacides peut en outre comprendre un ou plusieurs polyacides additionnels, distincts de l'acide succinique et de l'acide sébacique. Selon un mode de réalisation particulier, le ou les polyacides additionnels comprennent 2 ou 3 fonctions acides carboxyliques. Plus particulièrement, le mélange de polyacides comprend en outre l'acide adipique, l'acide azélaïque, l'acide citrique, l'acide téréphtalique, l'acide 2,5-furanedicarboxylique, l'acide fumarique, l'acide itaconique, l'acide muconique, et leurs mélanges. De préférence, le mélange de polyacides comprend en outre l'acide 2,5-furanedicarboxylique, l'acide citrique, et leurs mélanges.

Le mélange de polyacides (acide succinique, acide sébacique et éventuellement polyacide additionnel) peut notamment représenter de 35 à 70%, de préférence de 40 à 65%, plus préférentiellement de 45 à 60%, en moles des monomères incorporés dans la résine polyester.

L'acide succinique peut notamment représenter de 5 à 50%, de préférence de 10 à 45%, plus préférentiellement de 15 à 40%, en moles des monomères incorporés dans la résine polyester.

L'acide sébacique peut notamment représenter de 2 à 25%, de préférence de 5 à 20%, plus préférentiellement de 10 à 15%, en moles des monomères incorporés dans la résine polyester.

Le ratio molaire entre l'acide succinique et l'acide sébacique incorporés dans la résine polyester peut notamment être supérieur ou égal à 1, en particulier de 1 à 6, plus particulièrement 1 à 4.

Le ou les polyacides additionnels peuvent notamment représenter de 0 à 30%, de préférence de 2 à 25%, plus préférentiellement de 5 à 20%, en moles des monomères incorporés dans la résine polyester.

Selon un mode de réalisation particulier, le ratio molaire entre les diols (isosorbide et diol ramifié en C3-C6) et les polyacides (acide succinique, acide sébacique et éventuellement polyacide additionnel) est inférieur ou égal à 1,1, en particulier de 0,4 à 1, plus particulièrement de 0,6 à 1, plus particulièrement encore de 0,8 à 1.

Les quantités de chaque monomère sont ajustées afin d'obtenir une résine ayant masse molaire moyenne en nombre et une température de transition vitreuse adéquate pour l'application visée.

La résine polyester selon l'invention peut notamment présenter une masse molaire moyenne en nombre (Mn) de 1 000 à 3 500 g.mol⁻¹, de préférence de 1 100 à 3 000 g.mol⁻¹, plus préférentiellement de 1 200 à 2 500 g.mol⁻¹. La résine polyester selon l'invention peut notamment présenter une masse molaire moyenne en poids (Mw) de 1 000 à 8 000 g.mol⁻¹, de préférence de 1 500 à 7 000 g.mol⁻¹, plus préférentiellement de 2 500 à 6 000 g.mol⁻¹. La Mn et la Mw peuvent être mesurées selon la méthode décrite ci-après.

La résine polyester selon l'invention peut notamment présenter une température de transition vitreuse (Tg) de -30°C à 35°C, de préférence de -20°C à 25°C, plus préférentiellement de -10°C à 10°C. La Tg peut être mesurée selon la méthode décrite ci-après.

La résine polyester selon l'invention peut notamment présenter un indice d'acide de 40 à 150 mg KOH/g, de préférence de 45 à 125 mg KOH/g, plus préférentiellement de 50 à 100 mg KOH/g. L'indice d'acide peut être mesuré selon la méthode décrite ci-après. L'indice d'acide de la résine peut être contrôlé en fonction de l'avancée de la réaction de polymérisation. De préférence, la polymérisation est arrêtée avant que la réaction d'estérification soit totale afin de conserver des groupes acides carboxyliques libres dans la résine. Sans vouloir être lié par une quelconque théorie, la présence de groupes acides carboxyliques libres dans la résine permet d'améliorer l'interaction entre la résine et les ongles.

La résine peut être obtenue selon le procédé de préparation décrit ci-après.

### Procédé de préparation de la résine polyester

Le procédé de préparation de la résine polyester selon l'invention comprend une succession d'étapes d'estérification permettant d'obtenir une résine soluble incorporant une quantité élevée d'isosorbide.

Dans l'étape i), l'isosorbide réagit avec un polyacide choisi parmi l'acide succinique ou l'acide sébacique.

Dans l'étape ii), le mélange obtenu à l'étape i) réagit avec un diol ramifié en C3-C6 et un polyacide choisi parmi l'acide succinique ou l'acide sébacique.

Le polyacide utilisé dans l'étape i) est différent du polyacide utilisé dans l'étape ii). Ainsi, si l'isosorbide réagit avec l'acide succinique dans l'étape i), le mélange obtenu à l'étape i) réagira avec un diol ramifié en C3-C6 et l'acide sébacique. Alternativement, si l'isosorbide réagit avec l'acide sébacique dans l'étape i), le mélange obtenu à l'étape i) réagira avec un diol ramifié en C3-C6 et l'acide succinique.

Le ou les polyacides additionnels peuvent être ajoutés dans l'étape i) ou ii). En particulier, le ou les polyacides additionnels sont ajoutés dans l'étape i).

Les quantités d'isosorbide, de diol ramifié en C3-C6, d'acide sébacique, d'acide succinique et de polyacide additionnel introduits dans les étapes i) et ii) du procédé sont telles que définies précédemment pour la résine. En effet, le procédé selon l'invention permet une incorporation quasi-totale des monomères dans la résine polyester.

Le ratio final molaire entre les diols (isosorbide et diol ramifié en C3-C6) et les polyacides (acide succinique, acide sébacique et éventuellement polyacide additionnel) incorporés dans la résine polyester est avantageusement inférieur ou égal à 1,1, en particulier de 0,4 à 1, plus particulièrement de 0,6 à 1, plus particulièrement encore de 0,8 à 1.

Selon un mode de réalisation particulier, le ratio molaire entre l'isosorbide et les polyacides (acide succinique ou acide sébacique et éventuellement polyacide additionnel) introduits dans l'étape i) est de 0,2 à 1,1, plus particulièrement de 0,3 à 1, plus particulièrement encore de 0,4 à 1.

Selon un mode de réalisation particulier, le ratio molaire entre le diol ramifié en C3-C6 et les polyacides (acide succinique ou acide sébacique et éventuellement polyacide additionnel) introduits dans l'étape ii) est de 0,2 à 3, plus particulièrement de 0,4 à 2,5, plus particulièrement encore de 0,6 à 2.

Selon un mode de réalisation particulier, les étapes i) et ii) du procédé sont réalisées en l'absence de solvant organique.

Selon un mode de réalisation particulier, les étapes i) et ii) du procédé sont réalisées en l'absence de de catalyseur.

Les étapes i) et ii) peuvent notamment être réalisées sous atmosphère inerte. La réalisation de l'étape i) sous atmosphère inerte est notamment importante pour éviter la thermo-oxydation de l'isosorbide.

Les réactions d'estérification peuvent être accélérées en chauffant le milieu réactionnel. Les étapes i) et ii) peuvent notamment être réalisées à une température de 120°C à 200°C.

La réaction peut notamment être suivie en contrôlant l'indice d'acide à chaque étape. Une fois l'indice d'acide ciblé atteint, la température du milieu réactionnel peut être abaissée.

Le procédé de l'invention peut comprendre une étape iii) de dilution de la résine obtenue par ajout d'un solvant organique. Cette étape permet notamment de solubiliser la résine ce qui facilite sa mise en œuvre ultérieure.

Le solvant organique peut être tel que décrit ci-après dans la composition filmogène.

Le solvant organique peut être ajouté à la résine en une quantité suffisante pour d'obtenir une composition ayant un extrait sec en poids de 60 à 80%, en particulier 65 à 75%, plus particulièrement environ 70%.

La résine obtenue après dilution dans un solvant peut être mélangée avec un polymère filmogène cellulosique pour former une composition filmogène telle que décrite ci-après.

### Composition filmogène

La composition filmogène selon l'invention comprend la résine polyester de l'invention, un polymère filmogène cellulosique, et un solvant organique.

Par « composition filmogène » on entend une composition apte à former un film après application sur une surface, notamment sur la peau ou les matières kératiniques tels que les ongles.

Selon un mode de réalisation particulier, la composition filmogène comprend 1 à 25%, de préférence 4 à 15%, plus préférentiellement 5 à 10%, en poids de résine polyester par rapport au poids de la composition.

Le polymère filmogène cellulosique peut notamment être choisi parmi la nitrocellulose, l'acétobutyrate de cellulose, l'éthylcellulose, l'hydroxypropylcellulose et leurs mélanges. De préférence, le polymère filmogène cellulosique est la nitrocellulose.

Selon un mode de réalisation particulier, la composition filmogène comprend 1 à 25%, de préférence 6 à 20%, plus préférentiellement 10 à 15%, en poids de polymère filmogène cellulosique par rapport au poids de la composition.

Le solvant organique est avantageusement cosmétiquement acceptable, c'est-à-dire qu'il ne procure pas de désagréments (rougeurs, picotements) lorsqu'il est appliqué sur la peau et les matières kératiniques. Le solvant organique peut notamment être choisi parmi les composés aliphatiques, tels que les acétates, les cétones, les alcools, les alcanes, ou un mélange de ceux-ci. En particulier le solvant est choisi parmi l'acétate d'éthyle, l'acétate de butyle, l'acétate de propyle, l'acétate d'isobutyle, l'éthanol, le propanol, l'isopropanol, le butanol, la méthyléthylcétone, la méthylisobutylcétone, l'heptane, l'hexane, et des mélanges de ceux-ci. Plus particulièrement, le solvant organique est choisi parmi l'acétate d'éthyle, l'acétate de butyle, et leurs mélanges. Plus particulièrement encore, le solvant organique est un mélange d'acétate d'éthyle et d'acétate de butyle.

Selon un mode de réalisation préféré, la composition filmogène est anhydre, c'est-à-dire qu'elle ne comprend pas d'eau.

Selon un mode de réalisation particulier, la composition filmogène comprend 50 à 98%, de préférence de 65 à 90%, plus préférentiellement de 75 à 85%, en poids d'un solvant organique par rapport au poids de la composition.

La composition filmogène selon l'invention peut être introduite dans un vernis à ongles tel que décrit ci-après.

### Vernis à ongles

Le vernis à ongles selon l'invention comprend:
- la résine polyester selon l'invention telle que définie précédemment ou telle que préparée selon le procédé décrit précédemment, un polymère filmogène cellulosique, et un solvant organique telle que décrite précédemment ; et
- un composé choisi parmi un plastifiant, une deuxième résine, un agent rhéologique, une matière colorante, un additif et les mélanges de ceux-ci.

Le polymère filmogène cellulosique, le solvant organique et la teneur en résine polyester selon l'invention sont tels que définis ci-dessus par rapport à la composition filmogène.

Le plastifiant peut notamment permettre de moduler la dureté du film formé afin d'obtenir les caractéristiques physico-chimiques du film souhaitées. Des exemples de plastifiants sont l'acétyl tributyl citrate, l'acétyl triéthyl citrate, le tributyl citrate, le triéthylcitrate, le dibutyl phtalate, le triphénylphosphate, la triacétine, le triméthyl pentanyl diisobutyrate, la triéthylhexanoïne, le sucrose benzoate, le dibutyl adipate, le diéthyl phthalate, le diisobutyl adipate, le diisopropyl adipate et le dipropylène glycol dibenzoate.

Selon un mode de réalisation particulier, le plastifiant représente 2 à 12%, en particulier 4 à 10%, plus particulièrement 5 à 8%, en poids par rapport au poids du vernis à ongles.

La deuxième résine peut notamment permettre d'améliorer la brillance et l'adhésion du vernis sur l'ongle. La deuxième résine est distincte de la résine polyester selon l'invention. Des exemples de deuxième résines sont une résine tosylamide/formaldéhyde (Ketjenflex^{®} MH ou Ketjenflex^{®} MS-80 commercialisées par Akzo Nobel ou Sulfonex^{®} M-80 commercialisée par Estron), une résine tosylamide/époxy (Lustrabrite^{®} S ou Lustrabrite^{®} S-70 ou Nagellite^{®} 3050 commercialisées par Telechemische ou Polytex^{®} E-100 ou Polytex^{®} NX-55 commercialisées par Estron), un copolymère acide adipique/néopentyl glycol/anhydride triméllitique (Uniplex^{®} 670-P commercialisé par Unitex) un copolymère d'anhydride phtalique/glycérine/glycidyl décanoate, un copolymère d'anhydride phtalique/anhydride trimellitique/glycols (Polynex^{®} commercialisé par Estron), un copolymère de glycérine/acide phtalique, un copolymère styrène/acrylate/acrylonitrile, un polyacrylate, un copolymère d'acrylates, un copolymère d'acrylate et de styrène, le sucrose acétate isobutyrate (SAIB), un polyvinylbutyral et une résine de colophane (appelée aussi « rosin ») obtenue par distillation de la résine de pin.

Selon un mode de réalisation particulier, la deuxième résine représente 0,1 à 15%, en particulier 0,5 à 12%, plus particulièrement 1 à 10% et plus particulièrement encore de 1 à 7% en poids par rapport au poids du vernis à ongles.

L'agent rhéologique peut notamment permettre d'ajuster la viscosité du vernis à ongles et maintenir les particules insolubles, notamment les matières colorantes, en suspension dans le vernis. Des exemples d'agents rhéologiques sont les argiles, notamment une hectorite ou une bentonite, et des oxydes de silice.

Selon un mode de réalisation particulier, l'agent rhéologique représente 0,1 à 3%, en particulier 0,2 à 2%, plus particulièrement 0,5 à 1%, en poids par rapport au poids du vernis à ongles.

Une matière colorante peut notamment apporter de la couleur au vernis à ongles Des exemples de matières colorantes sont les pigments, les colorants solubles, les nacres, les paillettes et les particules métalliques.

Selon un mode de réalisation particulier, la matière colorante représente 0,1 à 20%, en particulier 0,5 à 12%, plus particulièrement 5 à 10%, en poids par rapport au poids du vernis à ongles.

Un additif permet de conférer une propriété spécifique au vernis à ongles. On peut ainsi citer les antioxydants qui permettent de protéger la formulation, notamment les matières colorantes, des rayons UV, des agents de surface ou encore des agents traitants pour renforcer les ongles, notamment la kératine, les vitamines, les acides aminés, et les alpha-hydroxyacides.

Selon un mode de réalisation particulier, l'additif représente 0,01 à 5%, en particulier 0,2 à 2%, plus particulièrement 0,5 à 1%, en poids par rapport au poids du vernis à ongles.

### Utilisation

La résine polyester selon l'invention est une résine biosourcée qui permet de conférer brillance et dureté à un vernis à ongles.

Ainsi, la présente invention vise à protéger l'utilisation de la résine polyester selon l'invention en tant que liant dans un vernis à ongles.

La présente invention est illustrée plus en détails dans les exemples non limitatifs décrits ci-après.

### Exemples

### Méthodes de mesure

### Mesure des masses molaires

Les analyses SEC ont été réalisées sur un chromatographe Alliance^{®} e2695 (Waters) à 40°C avec le THF comme éluant à un débit de 1 ml.min⁻¹ et avec des concentrations d'échantillon de 10 à 15 mg.mL⁻¹. Ce chromatographe était équipé d'un réfractomètre Optilab^{®} T-rEX (Wyatt Technology) et d'un set de colonnes HR1, HR2 et HR4 (Styragel). Enfin, une courbe de calibration des masses molaires a été réalisée avec des références polystyrène monodisperses.

### Mesure de la conversion des monomères

La conversion des monomères a été déterminée en mesurant le taux de monomères résiduels dans le polymère final. Ce dernier a été déterminé en utilisant un chromatographe 430-GC (Varian) équipé d'un port d'injection split/splitless (ratio 50:1), un détecteur à ionisation de flamme et une colonne ZB-5ms (Zebron). Une chauffe de 50°C à 250°C à la vitesse de chauffe de 10°C.min⁻¹ a été appliquée au four. Avant d'être injectés, les polymères ainsi que l'étalon interne (octanol) ont été silylés avec le N,O-Bis(triméthylsilyl)trifluoroacétamide en présence d'1% de chlorure de triméthylsilyle.

L'incorporation des monomères a été déterminée par RMN 1H. Les spectres ont été réalisés sur un spectromètre Bruker Avance I 300MHz à température ambiante dans le CDCI3 ou DMSO. Tous les spectres ont été obtenus après seize accumulations.

### Mesure de la température de transition vitreuse (Tg)

Les propriétés thermiques des résines ont été mesurées par calorimétrie différentielle à balayage sur un appareil DSC Q20 (TA instruments). Les échantillons ont été refroidis une première fois à -90°C pendant 10 min. Puis une première chauffe a été réalisée jusqu'à 115°C à la vitesse de 10°C.min⁻¹ et les échantillons ont été laissés à cette température pendant 5 min. Un refroidissement a ensuite été réalisé jusqu'à -90°C à la même vitesse et une seconde chauffe identique à la première a été appliquée. La Tg est mesurée lors de la seconde chauffe.

### Mesure de l'indice d'acide

L'indice d'acide a été mesuré par dosage selon la norme ISO 2114:2000 avec une solution méthanolique d'hydroxyde de potassium à 0.5 mol.L⁻¹ et avec la phénolphtaléine comme indicateur coloré. Les échantillons ont été dissouts dans un mélange diméthtylsulfoxide/dichlorométhane et la solution a été titrée jusqu'à l'obtention d'un changement de couleur persistant.

### Mesure de la brillance

Des revêtements d'épaisseur de 100 µm ont été réalisés sur une carte de contraste et séchés à l'étuve à 50°C pendant 15-30 min. La brillance a ensuite été mesurée avec un brillancemètre micro-TRI-gloss (BYK-Gardner) à 60° selon la norme ISO 2813:2014.

### Mesure de la dureté

Des revêtements d'épaisseur de 100 µm ont été appliqués sur des plaques de verre et séchés à température ambiante pendant 24h. La dureté a ensuite été mesurée avec un pendule de Persoz (Brandt) selon la norme ISO 1522:2006.

### Mesure de l'adhésion

Des revêtements d'épaisseur de 100 µm ont été appliqués sur des plaques de verre et séchés à température ambiante pendant 24h. L'adhésion a ensuite été mesurée avec le test du quadrillage selon la norme ISO 2409:2013. Dans ce test, les revêtements ont été découpés selon un quadrillage de 25 cases à l'aide d'un peigne (Byk-Gardner). Du scotch normé est ensuite appliqué sur le revêtement puis retiré. Les sections découpées sont examinées afin de vérifier la proportion de revêtement décollé. Une note de 0 à 5 est ensuite attribuée à l'adhésion du revêtement, 0 étant une adhésion parfaite c'est-à-dire aucune section n'a été décollée et 5 lorsque la totalité du revêtement a été décollée.

### Mesure du temps de séchage

Des revêtements d'épaisseur de 100 µm ont été réalisés sur une carte de contraste et séchés à température ambiante. Le temps de séchage a été mesuré avec le rhéolaser COATING (Formulaction). Le séchage sec au toucher a été choisi comme temps de séchage caractéristique par sa facilité à l'identifier sur la courbe.

### Exemple 1 : Préparation d'une résine polyester selon l'invention

De l'isosorbide (20,09 g, 137,5 mmol), de l'acide sébacique (11,12 g, 55 mmol) et de l'acide citrique (15,86 g, 82,6 mmol) ont été introduits dans un réacteur de 100 mL mis sous atmosphère inerte. Le réacteur a ensuite été chauffé à 120°C à l'aide d'un chauffe-ballon et agité par agitation mécanique à 200 tours/min. Une fois le milieu fondu (environ 80°C), 5-6 cycles de désoxygénation (azote-vide) ont été réalisés. La température a ensuite été progressivement augmentée jusqu'à 200°C. La réaction a été suivie par indice d'acide (IA). Une fois l'indice d'acide ciblé atteint (IA = 110 mg KOH/g), la température a été abaissée à 130°C. Le 1,2-propanediol (8,30 g, 109,1 mmol) et l'acide succinique (19,40 g, 164,3 mmol) ont été ajoutés au réacteur. La température a ensuite été augmentée à 190°C et la réaction suivie par indice d'acide jusqu'à atteindre un indice d'acide de 50 à 100 mg KOH/g. Ensuite la température a été abaissée à 120°C et la résine a été diluée avec 30% en poids d'un mélange d'acétate de butyle et d'acétate d'éthyle (23 g, 50/50 massique).

### Exemple 2 : Préparation d'une résine polyester selon l'invention

De l'isosorbide (18,27 g, 125 mmol), de l'acide sébacique (15,17 g, 75 mmol), de l'acide 2,5-furanedicarboxylique (FDCA) (3,90 g, 25 mmol) et de l'acide citrique (14,41 g, 75 mmol) ont été introduits dans un réacteur de 100 mL. Le réacteur a ensuite été chauffé à 120°C à l'aide d'un chauffe-ballon. Une fois le milieu majoritairement fondu (environ 110°C), 5-6 cycles de désoxygénation (azote-vide) ont été réalisés. La température a ensuite été progressivement augmentée jusqu'à 200°C. La réaction a été suivie par indice d'acide. Une fois l'indice d'acide ciblé atteint (IA = 210 mg KOH/g), la température a été abaissée à 140°C. Le 1,2-propanediol (9,50 g, 124,9 mmol) et l'acide succinique (8,84 g, 74,9 mmol) ont été ajoutés au réacteur. La température a ensuite été augmentée à 200°C et la réaction suivie par indice d'acide jusqu'à atteindre un indice d'acide de 50 à 100 mg KOH/g. Ensuite la température a été abaissée à 120°C et la résine a été diluée avec 30% en poids d'un mélange d'acétate de butyle et d'acétate d'éthyle (24 g, 50/50 massique).

### Exemple 3 : Préparation d'une résine polyester selon l'invention

De l'isosorbide (18,27 g, 125 mmol), de l'acide sébacique (15,17 g, 75 mmol) et de l'acide citrique (14,41 g, 75 mmol) ont été introduits dans un réacteur de 100 mL. Le réacteur a ensuite été chauffé à 120°C à l'aide d'un chauffe-ballon. Une fois le milieu majoritairement fondu (environ 80-90°C), 5-6 cycles de désoxygénation (azote-vide) ont été réalisés. La température a ensuite été progressivement augmentée jusqu'à 200°C. La réaction a été suivie par indice d'acide. Une fois l'indice d'acide ciblé atteint (IA = 160 mg KOH/g), la température a été abaissée à 140°C. Le 1,2-propanediol (9,50 g, 124,9 mmol) et l'acide succinique (11,80 g, 99,9 mmol) ont été ajoutés au réacteur. La température a ensuite été augmentée à 190°C et la réaction suivie par indice d'acide jusqu'à atteindre un indice d'acide de 50 à 100 mg KOH/g. Ensuite la température a été abaissée à 120°C et la résine a été diluée avec 30% en poids d'un mélange d'acétate de butyle et d'acétate d'éthyle (23 g , 50/50 massique).

### Exemple 4 : Préparation d'une résine polyester selon l'invention

De l'isosorbide (24,13 g, 165,1 mmol) et de l'acide succinique (25,98 g, 220 mmol) ont été introduits dans un réacteur de 100 mL. Le réacteur a ensuite été chauffé à 120°C à l'aide d'un chauffe-ballon. Une fois le milieu majoritairement fondu (environ 90°C), 5-6 cycles de désoxygénation (azote-vide) ont été réalisés. La température a ensuite été progressivement augmentée jusqu'à 200°C. La réaction a été suivie par indice d'acide. Une fois l'indice d'acide ciblé atteint (IA = 140 mg KOH/g), la température a été abaissée à 140°C. Le 1,2-propanediol (8,38 g, 110,1 mmol) et l'acide sébacique (11,12 g, 55 mmol) ont été ajoutés au réacteur. La température a ensuite été augmentée à 190°C et la réaction suivie par indice d'acide jusqu'à atteindre un indice d'acide de 50 à 100 mg KOH/g. Ensuite la température a été abaissée à 120°C et la résine a été diluée avec 30% en poids d'un mélange d'acétate de butyle et d'acétate d'éthyle (21 g, 50/50 massique).

### Contre-exemple 1 : Préparation d'une résine polyester non selon l'invention

De l'isosorbide (30,90 g, 211,4 mmol), de l'acide sébacique (11,41 g, 56,4 mmol), de l'acide 2,5-furanedicarboxylique (FCDA) (3,67 g, 23,5 mmol), de l'acide citrique (16,25 g, 84,6 mmol) et du 1,2-propanediol (7,21 g, 94,8mmol) ont été introduits dans un réacteur de 100 mL. Le réacteur a ensuite été chauffé à 120°C à l'aide d'un chauffe-ballon. Une fois le milieu majoritairement fondu (environ 110°C), 5-6 cycles de désoxygénation (azote-vide) ont été réalisés. La température a ensuite été progressivement augmentée jusqu'à 200°C. La réaction a été suivie par indice d'acide. Une fois l'indice d'acide ciblé atteint (IA=50-100 mg KOH/g), la température a été abaissée à 120°C et la résine a été diluée avec 30% en poids d'acétate de butyle (23 g). Cependant, la résine n'était pas soluble dans le solvant et précipitait à température ambiante. Ceci était dû à la faible incorporation de l'isosorbide (environ 70-75%) et de l'acide 2,5-furanedicarboxylique.

### Exemple 5 : Propriétés des résines polyester selon l'invention

Les résines obtenues dans les exemples 1-4 ont été caractérisées par leur masse molaire Mn et Mw, leur indice d'acide et leur Tg selon les méthodes de mesure décrites précédemment. Les résines selon l'invention sont comparées avec d'autres résines. Les résines CEx 1 à CEx 4 sont des résines pétrosourcées respectivement commercialisés sous les références Coverpol 1311 (Mäder), Coverpol 1312 (Mäder), TSER (Polytex E 75 par Estron) et TSFR (Lustralite 44.444 par DIC). La résine CEx 5 est une résine biosourcée commercialisée sous la référence Natipol 1303 (Mäder). Les résultats sont indiqués dans le tableau ci-dessous.

**[Tableau 1]**

| Résine | Isosorbide | 1,2-propanediol | Acide citrique | Acide laurique | Acide succinique | Acide sébacique | FCDA | Mn | Mw | IA | Tg |
|---|---|---|---|---|---|---|---|---|---|---|---|
| | | | | | | | | (g.mol-1) | (g.mol-1) | (mg KOH/g) | (°C) |
| CEx 1 | - | - | - | - | - | - | - | 1540 | 6170 | 50 | -4 |
| CEx 2 | - | - | - | - | - | - | - | 1010 | 3920 | 55 | -2 |
| CEx 3 | - | - | - | - | - | - | - | 230 | 360 | - | 18 |
| CEx 4 | - | - | - | - | - | - | - | 730 | 490 | - | 15 |
| CEx 5 | - | 55,6 | 14,8 | 7,4 | 22,2 | - | - | 902 | 2603 | 46 | -32 |
| Ex 1 | 25 | 20 | 15 | - | 30 | 10 | - | 1290 | 5400 | 92 | 1 |
| Ex 2 | 25 | 25 | 15 | - | 15 | 15 | 5 | 1330 | 2730 | 64 | -8 |
| Ex 3 | 25 | 25 | 15 | - | 20 | 15 | - | 1810 | 4020 | 63 | -12 |
| Ex 4 | 30 | 20 | - | - | 40 | 10 | - | 1670 | 2940 | 63 | -8 |

Les résines selon l'invention présentent une masse molaire Mn de 1 200 à 2 000 g. mol-1, une masse molaire Mw de 2 500 à 6 000, un indice d'acide de 50 à 100 mg KOH/g et une Tg de -15 à 10°C.

### Exemple 6 : Composition filmogène

Les résines de l'exemple 5 ont été introduites dans une composition filmogène comprenant :
- 7% de résine ;
- 14% de nitrocellulose ;
- 79% d'un mélange acétate d'éthyle/acétate de butyle (50/50 vol) ;
les pourcentages étant des pourcentages en poids par rapport au poids de la composition.

La dureté, l'adhésion et la brillance des films formés ont été mesurées selon les méthodes de mesure décrites précédemment.

**[Tableau 2]**

| Résine | Isosorbide | 1,2-propanediol | Acide citrique | Acide laurique | Acide succinique | Acide sébacique | FCDA | Dureté | Adhésion | Briliance | Séchage |
|---|---|---|---|---|---|---|---|---|---|---|---|
| | | | | | | | | (s) | (0 à 5) | (%) | (min) |
| CEx 1 | - | - | - | - | - | - | - | 388 | 0 | 91,9 | 17 |
| CEx 2 | - | - | - | - | - | - | - | 398 | 0 | 92,8 | 12 |
| CEx 3 | - | - | - | - | - | - | - | 400 | 0 | 97,5 | 16 |
| CEx 4 | - | - | - | - | - | - | - | 394 | 0 | 96,9 | 17 |
| CEx 5 | - | 55,6 | 14,8 | 7,4 | 22,2 | - | - | 375 | 0 | 90,8 | 19 |
| Ex 1 | 25 | 20 | 15 | - | 30 | 10 | - | 373 | 0 | 85,5 | 15 |
| Ex 2 | 25 | 25 | 15 | - | 15 | 15 | 5 | 382 | 0 | 85,7 | 17 |
| Ex 3 | 25 | 25 | 15 | - | 20 | 15 | - | 384 | 0 | 88,4 | n.d. |
| Ex 4 | 30 | 20 | - | - | 40 | 10 | - | 394 | 0 | 85,8 | n.d. |

Les compositions comprenant les résines selon l'invention présentent une dureté, une adhésion et une brillance équivalentes à celles des formulations comparatives. Le temps de séchage des compositions des Exemples 1 et 2 selon l'invention est inférieur à celui de la formulation comprenant la résine biosourcée CEx 5.

### Application industrielle

Les présentes solutions techniques peuvent trouver à s'appliquer notamment dans la formulation de vernis à ongles biosourcés.

La présente divulgation ne se limite pas aux exemples de résine polyester et de composition filmogène décrits ci-avant, seulement à titre d'exemple, mais elle englobe toutes les variantes que pourra envisager l'homme de l'art dans le cadre de la protection recherchée.

## Revendications

1. Résine polyester obtenue par polymérisation :
- d'un mélange de diols comprenant l'isosorbide et un diol ramifié en C3-C6 ; et
- d'un mélange de polyacides comprenant l'acide succinique et l'acide sébacique.

2. Résine polyester selon la revendication 1, **caractérisée en ce que** le diol ramifié en C3-C6 est choisi parmi le 1,2-propanediol, le 1,3-butanediol, le 2,3-butanediol, le 1,2-pentanediol, et leurs mélanges, de préférence le 1,2-propanediol.

3. Résine polyester selon la revendication 1 ou 2, **caractérisée en ce que** le mélange de polyacides comprend en outre un ou plusieurs polyacides additionnels, en particulier choisis parmi l'acide adipique, l'acide azélaïque, l'acide citrique, l'acide téréphtalique, l'acide 2,5-furanedicarboxylique, l'acide fumarique, l'acide itaconique, l'acide muconique, et leurs mélanges, plus particulièrement choisis parmi l'acide 2,5-furanedicarboxylique, l'acide citrique, et leurs mélanges.

4. Résine polyester selon l'une quelconque des revendications 1 à 3, **caractérisée en ce que** le mélange de polyacides représente de 35 à 70%, de préférence de 40 à 65%, plus préférentiellement de 45 à 60%, en moles des monomères incorporés dans la résine polyester.

5. Résine polyester selon l'une quelconque des revendications 1 à 4, **caractérisée en ce que** le mélange de diols représente de 30 à 65%, de préférence de 35 à 60%, plus préférentiellement de 40 à 55%, en moles des monomères incorporés dans la résine polyester.

6. Résine polyester selon l'une quelconque des revendications 1 à 5, **caractérisée en ce qu'**elle présente un indice d'acide de 40 à 150 mg KOH/g, de préférence de 45 à 125 mg KOH/g, plus préférentiellement de 50 à 100 mg KOH/g.

7. Procédé de préparation d'une résine polyester comprenant les étapes suivantes :
i) réaction d'isosorbide et d'un polyacide choisi parmi l'acide succinique ou l'acide sébacique ;
ii) réaction du mélange obtenu à l'étape i) avec un diol ramifié en C₃-C₆ et un polyacide choisi parmi l'acide succinique ou l'acide sébacique,
dans lequel le polyacide utilisé dans l'étape i) est différent du polyacide utilisé dans l'étape ii).

8. Composition filmogène comprenant une résine polyester telle que définie à l'une quelconque des revendications 1 à 6 ou telle que préparée selon le procédé de la revendication 7, un polymère filmogène cellulosique, et un solvant organique.

9. Vernis à ongles comprenant :
- une résine polyester telle que définie à l'une quelconque des revendications 1 à 6 ou telle que préparée selon le procédé de la revendication 7, un polymère filmogène cellulosique, et un solvant organique ; et
- un composé choisi parmi un plastifiant, une deuxième résine, un agent rhéologique, une matière colorante, un additif et les mélanges de ceux-ci.

10. Utilisation de la résine polyester telle que définie selon l'une quelconque des revendications 1 à 6 ou telle que préparée selon le procédé de la revendication 7, en tant que liant dans un vernis à ongles.

## Patentansprüche

1. Polyesterharz, erhalten durch Polymerisation:
- einer Mischung von Diolen, umfassend Isosorbid und ein verzweigtes C3-C6-Diol; und
- einer Mischung von Polysäuren, umfassend Bernsteinsäure und Sebacinsäure.

2. Polyesterharz nach Anspruch 1, **dadurch gekennzeichnet, dass** das verzweigte C3-C6-Diol aus 1,2-Propandiol, 1,3-Butandiol, 2,3-Butandiol, 1,2-Pentandiol und deren Mischungen, bevorzugt 1,2-Propandiol, gewählt ist.

3. Polyesterharz nach Anspruch 1 oder 2, **dadurch gekennzeichnet, dass** das Polysäuregemisch ferner eine oder mehrere weitere Polysäuren enthält, insbesondere gewählt aus Adipinsäure, Azelainsäure, Zitronensäure, Terephthalsäure, 2,5-Furandicarbonsäure, Fumarsäure, Itaconsäure, Muconsäure und deren Mischungen, insbesondere gewählt aus 2,5-Furandicarbonsäure, Zitronensäure und deren Mischungen.

4. Polyesterharz nach einem der Ansprüche 1 bis 3, **dadurch gekennzeichnet, dass** die Mischung aus Polysäuren 35 bis 70 %, bevorzugt 40 bis 65 %, stärker bevorzugt 45 bis 60 % der in das Polyesterharz eingebauten Monomere ausmacht.

5. Polyesterharz nach einem der Ansprüche 1 bis 4, **dadurch gekennzeichnet, dass** die Diolmischung 30 bis 65 %, bevorzugt 35 bis 60 %, stärker bevorzugt 40 bis 55 % der in das Polyesterharz eingebauten Monomere ausmacht.

6. Polyesterharz nach einem der Ansprüche 1 bis 5, **dadurch gekennzeichnet, dass** es eine Säurezahl von 40 bis 150 mg KOH/g, bevorzugt von 45 bis 125 mg KOH/g, stärker bevorzugt von 50 bis 100 mg KOH/g aufweist.

7. Verfahren zur Herstellung eines Polyesterharzes, umfassend die folgenden Schritte:
i) Reaktion von Isosorbid und einer Polysäure, gewählt aus Bernsteinsäure oder Sebacinsäure;
ii) Reaktion des in Schritt i) erhaltenen Gemisches mit einem verzweigten Diol C3-C6 und einer Polysäure, gewählt aus Bernsteinsäure oder Sebacinsäure, wobei die in Schritt i) verwendete Polysäure sich von der in Schritt ii) verwendeten Polysäure unterscheidet.

8. Filmbildende Zusammensetzung, umfassend ein Polyesterharz, wie in einem der Ansprüche 1 bis 6 definiert oder nach dem Verfahren nach Anspruch 7 hergestellt, ein filmbildendes Cellulosepolymer und ein organisches Lösungsmittel.

9. Nagellack, umfassend:
- ein Polyesterharz, wie in einem der Ansprüche 1 bis 6 definiert oder nach dem Verfahren nach Anspruch 7 hergestellt, ein filmbildendes Cellulosepolymer und ein organisches Lösungsmittel; und
- eine Verbindung, gewählt aus einem Weichmacher, einem zweiten Harz, einem Rheologiemittel, einem Farbstoff, einem Additiv und Mischungen davon.

10. Verwendung des Polyesterharzes, wie in einem der Ansprüche 1 bis 6 definiert oder nach dem Verfahren nach Anspruch 7 hergestellt, als Bindemittel in einem Nagellack.

## Claims

1. Polyester resin obtained by polymerising:
- a mixture of diols comprising isosorbide and a branched C3-C6 diol; and
- a mixture of polyacids comprising succinic acid and sebacic acid.

2. Polyester resin according to claim 1, **characterised in that** the branched C3-C6 diol is selected from 1,2-propanediol, 1,3-butanediol, 2,3-butanediol, 1,2-pentanediol, and mixtures thereof, preferably 1,2-propanediol.

3. Polyester resin according to claim 1 or 2, **characterised in that** the mixture of polyacids further comprises one or more additional polyacids, in particular selected from adipic acid, azelaic acid, citric acid, terephthalic acid, 2,5-furanedicarboxylic acid, fumaric acid, itaconic acid, muconic acid, and mixtures thereof, more particularly selected from 2,5-furanedicarboxylic acid, citric acid, and mixtures thereof.

4. Polyester resin according to any one of claims 1 to 3, **characterised in that** the mixture of polyacids represents from 35 to 70%, preferably from 40 to 65%, more preferably from 45 to 60%, in moles of the monomers incorporated into the polyester resin.

5. Polyester resin according to any one of claims 1 to 4, **characterised in that** the mixture of diols represents from 30 to 65%, preferably from 35 to 60%, more preferably from 40 to 55%, in moles of the monomers incorporated into the polyester resin.

6. Polyester resin according to any one of claims 1 to 5, **characterised in that** it has an acid number of 40 to 150 mg KOH/g, preferably of 45 to 125 mg KOH/g, more preferably of 50 to 100 mg KOH/g.

7. Method for preparing a polyester resin comprising the following steps of:
i) reacting isosorbide and a polyacid selected from succinic acid or sebacic acid;
ii) reacting the mixture obtained in step i) with a branched C₃-C₆ diol and a polyacid selected from either succinic acid or sebacic acid,
wherein the polyacid used in step i) is different from the polyacid used in step ii).

8. Film-forming composition comprising a polyester resin as defined in any one of claims 1 to 6 or as prepared according to the method of claim 7, a cellulosic film-forming polymer, and an organic solvent.

9. Nail polish comprising:
- a polyester resin as defined in any one of claims 1 to 6 or as prepared according to the method of claim 7, a cellulosic film-forming polymer, and an organic solvent; and
- a compound selected from a plasticiser, a second resin, a rheological agent, a colouring material, an additive, and mixtures thereof.

10. Use of the polyester resin as defined according to any one of claims 1 to 6 or as prepared according to the method of claim 7, as a binder in a nail polish.
